# EUROPÄISCHE PATENTSCHRIFT

(11) **EP 0 667 152 B1**
(45) Veröffentlichungstag und Bekanntmachung des Hinweises auf die Patenterteilung: **14.04.1999**
(21) Anmeldenummer: 95101172.5
(22) Anmeldetag: 27.01.1995
(51) Int. Cl.: A61K 31/19

(54) **Orale Verwendung von Calciumformiat zur Behandlung von Calciumdefizienz bei Tieren**
Oral use of calcium formate for the treatment of calcium deficiency in animals
Utilisation orale de formiate de calcium pour le traitement de carence à calcium chez des animaux

(30) Priorität: 28.01.1994 DE 4402544
(43) Veröffentlichungstag der Anmeldung: 16.08.1995
(73) Patentinhaber: Chevita GmbH, D-85276 Pfaffenhofen/Ilm (DE)
(72) Erfinder: Abele, Ulf, Dr., D-80804 München (DE)
(74) Vertreter: Jaeger, Klaus, Dr.

(56) Entgegenhaltungen:
- DE-A- 2 161 897
- VET. RECORD, Bd. 101, Nr. 20, 1977 Seiten 405-407, P.A. MULLEN 'Milk fever: a case against polypharmacy solutions.'

## Beschreibung

Die Erfindung betrifft die Verwendung von Calciumformiat zur Herstellung von oral verabreichbaren Zusammensetzungen nach dem Patentanspruch 1.

Es ist bekannt, Calciumsalz enthaltende Lösungen für die Herstellung von oral verabreichbaren Mitteln für Therapie, Prophylaxe und Metaphylaxe von Calciummangelerscheinungen bei Kühen, insbesondere bei Milchfieber, einzusetzen. Hier werden insbesondere solche Calciumverbindungen eingesetzt, die gut wasserlöslich und schnell aus dem Verdauungstrakt resorbierbar sind.

Ein nicht unerheblicher Anteil aller Kühe erkrankt unmittelbar nach dem Kalben bzw. mit Beginn der Laktation an Milchfieber (Gebärparese), ausgelöst durch erheblichen Calciummangel im Blut der Tiere.

Bekannt ist, diese Krankheit durch intravenöse Injektionen von Calcium, insbesondere in Form von Calciumborongluconat, zu behandeln. Da die intravenöse Injektion von Calcium gerade bei Rindern aufwendig ist und diese Krankheit bei einmal erkrankten Tieren zyklisch wieder auftritt, ist es auch bekannt, über orale Verabreichung von Calciumverbindungen in gelöster Form, insbesondere auch eines leicht resorbierbaren Calciumsalzes, diese Krankheit zu therapieren bzw. in Prophylaxe diese leicht resorbierbaren Calciumpräparate zu verabreichen.

Diese Calciumpräparate werden dem Tier in der Regel als hochkonzentrierte Lösung von Calciumchlorid verabreicht, besser gesagt, dem Tier über das Maul eingeschüttet. Der schlechte, bitter bis scharf brennende Geschmack und lokale Irritationen sowie Schleimhautverätzungen durch diese Calciumchloridlösung haben dazu geführt, daß sich die Tiere weigern, diese Präparate aufzunehmen. Dies birgt die Gefahr in sich, daß diese sehr ätzende Lösung statt in den Verdauungstrakt der Tiere durch Verschlucken in die Luftröhre bzw. in die Lunge gelangt, was zur Verätzung der Lungen bzw. in schlimmen Fällen sogar bis zum Tod führen kann.

Dies macht es praktisch nicht möglich, diese Methode der Verabreichung von Calciumchloridlösung als generelle Vorsorgemaßnahme gegen Calciummangel, insbesondere zur Prävention von Milchfieber, ohne tierärztliche Kontrolle anzuwenden.

Trotz dieser Nachteile, die diese leicht viskose Calciumchloridlösung aufweist, wurde sie über viele Jahre verwendet. Das Problem des Verschluckens der Flüssigkeit in Luftröhre und Lunge kann wesentlich reduziert werden, wenn die Calciumchloridlösung als Gel auf Basis von Wasser und einem inerten Cellulosederivat verabreicht wird.

Da alle bekannten, vorab genannten Verabreichungsformen, in denen Calciumchlorid, wenn auch in Gel eingebunden, verabreicht wird, Probleme bei der Aufnahme machen, da die Präparate bitter bis scharf brennend und daher schlecht schmecken und teilweise sogar Verätzungen des Verdauungstraktes bis hinein in den Pansen verursachen, wurde nach einer Verabreichungsform gesucht, die insbesondere geschmacklich verbessert ist und bei welcher die Gefahr des Verätzens und die Gefahr lokaler Irritationen stark gemindert wird.

Bekannt ist es, gelöstes Calciumsalz in einer Wasser-in-Öl-Emulsion einzubinden, wobei ein Calciumsalz in der wäßrigen Phase dieser Wasser-in-Öl-Emulsion gelöst ist und die ölige Phase aus einem zur Fütterung geeigneten Öl besteht. Auch hier wurde auf die gut lösliche Calciumkomponente Calciumchlorid zurückgegriffen. Als geeignetes Öl kann Pflanzenöl, beispielsweise Sonnenblumenöl, Erdnußnöl, u.a. verwendet werden. Aber auch tierische Öle, wie beispielsweise Fischöl, können für diese ölige Phase Verwendung finden. Obwohl diese Wasser-Öl-Emulsion nicht mehr so schlecht schmeckend, weniger stark ätzend und milder ist als eine reine Calciumchloridlösung, die bisher bei Milchfieber verabreicht wurde, können auch diese Wasser-in-Öl-Emulsionen bisweilen klinische Symptome hervorrufen und verursachen trotzdem, wenn auch geringe, pathologische Gewebsveränderungen im Magen-Darm-Trakt der Tiere sowie ein gewisses Abwehrverhalten der Tiere.

Bei den bisher bekannten Präparaten mit Calciumchlorid liegt die zu verabreichende Einzeldosis zwischen 370 und 500 ml mit einem Calciumgehalt (Ca++) von ca. 50g. Da für die Verabreichung ein möglichst kleines Volumen anzustreben ist, schien bisher für diesen Anwendungsbereich als Calciumverbindung nur Calciumchlorid in Betracht zu kommen, da dieses einerseits einen hohen Calciumgehalt und andererseits eine ausgezeichnete Wasserlöslichkeit aufweist.

Ausgehend von diesem Stand der Technik ist es die Aufgabe der vorliegenden Erfindung, eine Calciumverbindung zu finden, die nicht so schlecht schmeckend ist, teilweise pathologische Gewebsirritationen im Magen-Darm Trakt der Tiere weitestgehend ausschließt und einen ausreichend hohen Calciumgehalt in einem relativ kleinem Darreichungsvolumen aufweist.

Die Erfindung beruht auf der überraschenden Erkenntnis, daß die vorab genannten Nachteile durch die Verwendung von Calciumformiat in oral verabreichbaren Zusammensetzungen zu Prophylaxe, Therapie und Metaphylaxe von Calciummangelerscheinungen vermieden werden können.

Es hat sich anhand von Vergleichsversuchen mit calciumchloridhaltigen Mitteln als Wasser-in-Öl-Emulsionen gezeigt, daß die Verabreichung von Calciumformiat insbesondere als wäßriges Gel zur Prophylaxe, Metaphylaxe und insbesondere zur Therapie von Milchfieber bei Kühen, gute Erfolge gebracht hat.

Die Resorption von Calcium aus Calciumformiat über den Verdauungstrakt ist vergleichbar gut der Resorption aus Calciumchlorid bisher bekannter Produkte. Im Gegensatz zu Calciumchlorid verursacht Calciumformiat jedoch keinerlei Verätzungen oder Irritationen der Schleimhäute im Verdauungstrakt. Durch den deutlich besseren Geschmack von Calciumformiat gegenüber Calciumchlorid wird das Präparat wesentlich besser von den Tieren aufgenommen, was ein erheblich geringeres Abwehrverhalten der Tiere beim Verabreichen zur Folge hat und die Arbeit des Landwirtes oder des Tierarztes sehr erleichtert.

Die Einbindung des Calciumformiates in ein Gel oder eine Paste einerseits und der verbesserte Geschmack des Calciumpräparates andererseits haben den Vorteil, daß dieses Präparat von den Tieren gut akzeptiert und aufgenommen wird und damit die gefürchtete Aspiration in die Luftwege bzw. die Lunge durch Verschlucken fast vollständig ausgeschlossen wird.

Die Erfindung beruht weiterhin auch noch auf der Erkenntnis, daß die verwendete Calciumverbindung nicht schon im Produkt vollständig als Lösung vorliegen muß. Es ist völlig ausreichend, wenn das Calciumformiat in einer im Produkt gleichmäßig verteilten Suspension vorliegt und erst im Verdauungstrakt in Lösung geht und somit resorbierbar und physiologisch verfügbar wird. Erfindungsgemäß wird dies gelöst, indem das Calciumformiat im Präparat nur zum geringen Teil gelöst, aber zum größeren Teil als gel- oder pastenförmige Suspension vorliegt, so daß sich diese nicht entmischen kann. Auf diese Weise ist es vorteilhafterweise möglich, das zu verabreichende Volumen bei einem noch fließfähigen, einschüttbaren Calciumformiatgel auf ca. 300 bis 350 ml und bei einer Calciumformiatpaste auf ca. 200 bis 250 ml bei einem Calciumgehalt von ca. je 50 g Ca++ zu reduzieren, was die Verabreichung zusätzlich sehr vereinfacht.

In das Gel bzw. in die Paste können neben dem Calciumformiat auch Kombinationen von Magnesium- und/oder Phosphor-Verbindungen eingebunden werden. Des weiteren können dieser Paste bzw. diesem Gel geschmacksverbessernde Zusatzstoffe beigefügt werden, die von den Tieren bevorzugt werden, so daß diese dieses Präparat ohne Zögern aufnehmen.

Als Gele, in welche das Calciumformiat eingebunden wird, können sowohl Oleogele als auch wäßrige Gele Anwendung finden.

Die Erfindung soll nachstehend an zwei Ausführungsbeispielen näher erläutert werden.

Als Ausführungsbeispiele werden Gele mit Calciumformiat als Ergänzungsfuttermittel zur Prophylaxe und Metaphylaxe von postnatalem Calciummangel (Milchfieber) bei Kühen, Schafen oder Ziegen aufgeführt.

### Ausführungsbeispiel 1:

Auch das zukünftig auf dem Markt erhältliche Produkt "Baymix^{(R)} Calform" kann erfindungsgemäß Anwendung finden. Dieses Produkt ist wie folgt zusammengesetzt:

| | |
|---|---|
| Ca(HCOO)₂ | 38,83 % |
| MgCl₂ x 6 H₂O | 00,96 % |
| Wasser | 59,36 % |
| Gelbildner und Aromastoffe | 0,85 % |

Dieses Produkt ist ein wäßriges Gel, welches leicht in das Tiermaul einschüttbar ist.

### Ausführungsbeispiel 2:

Dieses Produkt ist ein Oleogel als Paste, welches mittels Applikator in das Tiermaul verabreicht wird. Das Produkt ist wie folgt zusammengesetzt:

| | |
|---|---|
| Ca(HCOO)₂ | 55,40 % |
| Erdnußöl | 42,30 % |
| Aerosil | 00,90 % |
| Antioxidans | 00,10 % |
| Aromastoffe | 00,30 % |
| Hilfsstoffe | 01,00 %. |

## Patentansprüche

1. Verwendung von Calciumformiat zur Herstellung von oral verabreichbaren Zusammensetzungen zur Prophylaxe und Metaphylaxe von Calciummangel bei Tieren.

2. Verwendung nach Anspruch 1,
dadurch **gekennzeichnet,**
daß das Calciumformiat zur Herstellung von oral verabreibaren Zusammensetzungen zur Therapie bei Milchfieber bei Rindern eingesetzt wird.

3. Verwendung nach Anspruch 1 oder 2,
daß es sich bei den oral verabreichbaren Zusammensetzungen um ein Gel oder eine Paste handelt.

4. Verwendung nach einem der Ansprüche 1 bis 3,
dadurch **gekennzeichnet,**
daß die oral verabreichbaren Zusammensetzungen das Calciumformiat in Kombination mit Magnesium- und/oder Phosphorverbindungen aufweisen und ein Gel oder eine Paste darstellen.

## Claims

1. The use of calcium formate for producing orally administrable compositions for purposes of prophylaxis and metaphylaxis of calcium deficiency in animals.

2. Use according to claim 1,
**characterized** in that
the calcium formate is used for producing orally administrable composition for treating milk fever in cattles.

3. Use according to claim 1 or 2,
**characterized** in that
the orally administrable compositions are a gel or a paste.

4. Use according to one of the claims 1 to 3,
**characterized** in that
the orally administrable compositions contain the calcium formate in combination with magnesium or phosphorous compounds and represent a gel or a paste.

## Revendications

1. Utilisation du formiate de calcium pour la préparation de compositions pouvant être administrées par voie orale, destinées à la prophylaxie et à la métaphylaxie de la carence en calcium chez des animaux.

2. Utilisation selon la revendication 1, caractérisée en ce que le formiate de calcium est utilisé pour la préparation de compositions pouvant être administrées par voie orale, destinées au traitement de la fièvre vitulaire chez des bovins.

3. Utilisation selon la revendication 1 ou 2, caractérisée en ce que les compositions pouvant être administrées par voie orale consistent en un gel ou une pâte.

4. Utilisation selon les revendications 1 à 3, caractérisée en ce que les compositions pouvant être administrées par voie orale comportent le formiate de calcium en association avec des composés à base de phosphore et/ou de magnésium, et consistent en un gel ou en une pâte.
